# EUROPEAN PATENT APPLICATION

(11) **EP 4 082 428 A1**
(43) Date of publication of application: **02.11.2022**
(21) Application number: 20910968.5
(22) Date of filing: 22.02.2020
(51) Int. Cl.: A61B 5/0215, A61B 5/00

(54) **INTRAVASCULAR PRESSURE MEASUREMENT SYSTEM**

(30) Priority: 31.12.2019 CN 201911423088
(71) Applicant: Insight Lifetech Co., Ltd., Shenzhen, Guangdong 518000 (CN)
(72) Inventor: LI, Lianbo, Shenzhen, Guangdong 518101 (CN); ZHENG, Yuxiao, Shenzhen, Guangdong 518101 (CN)
(74) Representative: Isern Patentes y Marcas S.L.
(86) International application number: PCT/CN2020/076312
(87) International publication number: WO 2021/134868

(57) **Abstract**

The present disclosure relates to an intravascular pressure measurement system comprising: a first pressure measurement device having a first pressure sensor for measuring the pressure in a blood vessel and an X-ray opaque development ring provided on at least one side of the first pressure sensor; a pullback device connected to the first pressure measurement device and controlling the first pressure measurement device to be pulled back in the blood vessel, wherein during the pullback procedure, the first pressure sensor acquires the first pressure data in the blood vessel, the first pressure data comprising a first pressure waveform of a plurality of cardiac cycles; and a host configured to obtain a blood vessel X-ray angiographic images at the preset part and an X-ray real-time images, in the blood vessel at the preset part, of the first pressure measurement device containing the development ring, wherein the host is connected to the pullback device and configured to receive the first pressure data, and the host obtains the pressure distribution diagram of the blood vessel of the preset part based on the X-ray angiographic images, the X-ray real-time images, and the first pressure data.

## Description

### BACKGROUND OF INVENTION

### Field of Invention

The present disclosure specifically relates to an intravascular pressure measurement system.

### Description of Related Art

As coronary artery disease is one of the major causes of death worldwide, the capability of diagnosing, monitoring, and treating coronary artery disease in more effective way can save lives. Coronary angiography is a conventional technique used to evaluate stenotic lesions of coronary arteries, but coronary angiography cannot reflect the real situation of coronary vascular function; so it is basically impossible to determine whether coronary arteries of stenotic lesions are related to the symptom of myocardial ischemia of a patient or not. Currently, the method used to determine the stenotic lesion of coronary artery in clinical practice is mainly to use the technology of Fractional Flow Reserve (FFR) obtained by pressure guide wire examination.

However, the following defects exist in the process of obtaining FFR: maximal congestion-inducing drugs (e.g., adenosine, adenosine triphosphate, ATP) is needed to inject into the coronary artery prior to measuring FFR so that the coronary artery is in the state of maximum congestion, increases the clinical operation time, resulting in indications to the patient, significantly increasing the medical cost, as well as the patients' allergic reaction.

In addition, the FFR value obtained by the current intravascular pressure measurement system can only determine whether the blood flow transmission between two measurement points is blocked or not, but cannot give the blood flow transmission situation between the two measurement points. The clinical guiding significance of the FFR value obtained by the current intravascular pressure measurement system is little when there is a tandem lesion or diffuse lesion between two measurement points. Furthermore, the intravascular pressure measurement system in the prior art can only qualitatively determine whether a stent needs to be implanted or not, but and cannot give quantitative information such as the size of the part where the stent needs to be implanted.

### SUMMARY OF INVENTION

The present disclosure is made in view of the above-mentioned situation and its object is to provide an intravascular pressure measurement system capable of eliminating the need to inject the maximum congestion inducing drug and capable of more specifically determining the size of the part where the stent needs to be implanted.

For the above purpose, the present disclosure provides an intravascular pressure measurement system, characterized by comprising: a first pressure measurement device having a first pressure sensor for measuring the pressure in a blood vessel and an X-ray opaque development ring provided on at least one side of the first pressure sensor; a pullback device connected to the first pressure measurement device and controlling the first pressure measurement device to be pulled back in the blood vessel, wherein during the pullback, the first pressure sensor acquires a first pressure data in the blood vessel, the first pressure data comprising a first pressure waveform of a plurality of cardiac cycles; and a host configured to obtain blood vessel X-ray angiographic images at a preset part and X-ray real-time images in the blood vessel at the preset part of the first pressure measurement device containing the development ring, wherein the host is connected to the pullback device and configured to receive the first pressure data, and the host obtains the pressure distribution diagram of the blood vessel of the preset part based on the X-ray angiographic images, the X-ray real-time images, and the first pressure data.

In the present disclosure, the first pressure measurement device has a first pressure sensor used to measure the pressure in the blood vessel, the first pressure sensor is provided with an X-ray opaque development ring at least one side The pullback device is connected to the first pressure measurement device and controls the pullback of the first pressure measurement device within the blood vessel; during the pullback, the first pressure sensor acquires the first pressure data within the blood vessel, the pullback device is connected to the host, and the host is configured to receive the first pressure data. The host is configured to obtain the X-ray angiographic images of the blood vessel and the X-ray real-time images of the first pressure measurement device including the development ring within the blood vessel; the host obtains a pressure distribution diagram of the blood vessel at the preset part based on the X-ray angiographic images, the X-ray real-time images, and the first pressure data. In this case, the present disclosure enables the determination of a specific lesion condition of a blood vessel of a patient without requiring the injection of maximum congestion inducing drug.

In the intravascular pressure measurement system according to the present disclosure, optionally, the host sets a preset starting point and a preset ending point in the blood vessel based on the X-ray angiographic images, and the pullback device controls the first pressure measurement device to be pulled back from the preset starting point to the preset ending point. Therefore, the first pressure measurement device can be pulled back from the preset starting point to the preset ending point.

In the intravascular pressure measurement system of the present disclosure, optionally, the preset starting point is far away from an aortic port and the preset ending point is close to the aortic port. It is thereby able to measure the intravascular pressure of the coronary artery.

In the intravascular pressure measurement system of the present disclosure, optionally, the host determines whether pressure signals in the blood vessel are stable or not based on the first pressure waveform obtained by the first pressure measurement device, and controls the pullback device to pull when the pressure signals are stable. Therefore, the host can control the pullback device to pull.

In the intravascular pressure measurement system of the present disclosure, optionally, the system includes a second pressure measurement device, wherein the second pressure measurement device has a second pressure sensor for measuring a pressure of a proximal side in the blood vessel and generating a second pressure data, the second pressure data comprising a second pressure waveform of a plurality of cardiac cycles. The second pressure data can thus be obtained.

In the intravascular pressure measurement system of the present disclosure, optionally, the host receives the second pressure data, and the host obtains a plurality of target ratios based on the first pressure data and the second pressure data, and obtains a target ratio distribution map of a blood vessel at the preset part based on the X-ray angiographic images, the X-ray real-time images, and the multiple target ratios. The target ratio distribution map can thus be obtained.

In the intravascular pressure measurement system of the present disclosure, optionally, the host is configured to determine the plurality of cardiac cycles based on the first pressure waveform or the second pressure waveform, calculate a plurality of pressure ratios of pressure values of the first pressure waveform to the pressure values of the corresponding second pressure waveform, and sequentially in the order from small to large of the ratios of each cardiac cycle select an average value of a predetermined number of pressure ratios, , or a minimum value of the pressure ratios corresponding to a diastolic phase in each cardiac cycle. The target ratio can thus be obtained.

In the intravascular pressure measurement system of the present disclosure, optionally, the host is configured to determine the plurality of cardiac cycles based on the first pressure waveform or the second pressure waveform, calculate a curve of pressure ratios of pressure values of the first pressure waveform to pressure values of a corresponding second pressure waveform as a first changing curve, and obtain a curve of mean pressure values of the second pressure waveform based on the second pressure waveform as a second changing curve, wherein a point at which the second pressure waveform in the descending time intersects the second changing curve in each cardiac cycle is a first intersection point corresponding to the cardiac cycle; the pressure ratio corresponding to a minimum value of the second pressure waveform in the descending time in each of cardiac cycles, or the pressure ratio corresponding to a midpoint of a time period from the first intersection point to the minimum value of the second pressure waveform in the cardiac cycle, or an average pressure ratio value corresponding to the time period from the first intersection point in the cardiac cycle to 80% of the cardiac cycle, or the average pressure ratio value corresponding to the time period from the first intersection point to the minimum value of the second pressure waveform in the cardiac cycle is taken as target ratio. The target ratio can thus be obtained.

In the intravascular pressure measurement system of the present disclosure, optionally, the host is configured to determine a plurality of cardiac cycles based on the first pressure waveform or the second pressure waveform, calculate a curve of pressure ratios of pressure values of the first pressure waveform to the pressure values of a corresponding second pressure waveform as a first changing curve, and obtain the curve of derivatives of the pressure values of the first pressure waveform with respect to time based on the first pressure waveform as a third changing curve, wherein a moment corresponding to a midpoint of the time period from a maximum value of the first pressure waveform to a minimum value of the third changing curve in any cardiac cycle is a first midpoint moment, a moment corresponding to the midpoint of a time period from a maximum value of the first pressure waveform to a maximum value of the third changing curve in any cardiac cycle is a second midpoint moment, and a pressure ratio value corresponding to the midpoint of a time period from the first midpoint moment to the second midpoint moment in the cardiac cycle, or a average pressure ratio value corresponding to a time period from a first to a last occurrence of a target derivative value in a time period from the first midpoint moment to the second midpoint moment in the cardiac cycle is taken as the target ratio; wherein the target derivative value is a derivative value that occurs most frequently in the third changing curve during the time period from the first midpoint moment to the second midpoint moment in the cardiac cycle during the time period from the first midpoint moment to the second midpoint moment in the cardiac cycle. The target ratio can thus be obtained.

In the intravascular pressure measurement system of the present disclosure, optionally, the host adjusts a pullback speed of the pullback device. The subsequently obtained pressure distribution diagrams or target ratio distribution diagrams can thus be more detailed.

In the intravascular pressure measurement system of the present disclosure, optionally, the host obtains a pressure gradient diagram of a length direction of a blood vessel at a preset part based on the first pressure data and the pullback speed, the pressure gradient diagram being a change diagram as a pressure within the blood vessel changes along a length direction of the blood vessel. The pressure gradient diagram can thus be obtained.

In the intravascular pressure measurement system of the present disclosure, optionally, the host adjusts the pullback speed of the pullback device to reduce the pullback speed of the pullback device when the magnitude of the pressure in the pressure gradient diagram decreases with the length direction of the blood vessel by no less than a preset threshold value. The pressure gradient diagram obtained can thus be made more detailed.

In the intravascular pressure measurement system of the present disclosure, optionally, the host is configured to synchronize the first pressure waveform and the second pressure waveform such that a moment when a peak value of the first pressure waveform appears in the cardiac cycle is the same as the moment when the peak value of the second pressure waveform appears. The first pressure waveform and the second pressure waveform can thus be synchronized to facilitate the subsequent acquisition of an accurate target ratio.

According to the present invention, an intravascular pressure measurement system capable of eliminating the need to inject the maximum congestion inducing drug and capable of specifically determining the size of the part where the stent needs to be implanted can be provided.

### BRIEF DESCRIPTION OF THE DRAWINGS

Fig. 1 is a schematic view showing a structure of an intravascular pressure measurement system according to an example of the present disclosure.
Fig. 2 is a schematic view showing the structure of an intravascular pressure measurement system according to another example of the present disclosure.
Fig. 3 is a schematic view showing an intravascular pressure measurement system according to an example of the present disclosure.
Fig. 4 is a angiographic images showing a blood vessel of a preset part according to an example of the present disclosure.
Fig. 5 is a schematic view showing an intravascular pressure measurement system according to an example of the present disclosure intervening in a human body.
Fig. 6 is a cross-sectional view showing an intravascular pressure measurement system according to an example of the present disclosure applied to the blood vessel in Fig. 4.
Fig. 7 shows the pressure distribution diagram of the blood vessel of Fig. 4 according to an example of the present disclosure.
Fig. 8 is a diagram showing pressure changes for measuring a plurality of cardiac cycles of the blood vessel of Fig. 4 according to an example of the present disclosure.
Fig. 9 is a diagram showing the pressure gradient of the blood vessel of Fig. 4 according to an example of the present disclosure.
Fig. 10 is a diagram showing pressure changes for measuring a plurality cardiac cycles of the blood vessel of Fig. 4 according to another example of the present disclosure.
Fig. 11 is a pressure waveform diagram showing a plurality of cardiac cycles of Fig. 10 according to an example of the present disclosure.
Fig. 12 is a pressure waveform diagram showing one cardiac cycle of Fig. 10 according to another example of the present disclosure.

### DETAILED DESCRIPTION

Hereinafter, preferred embodiments of the present disclosure will be described in detail with reference to the accompanying drawings. In the following description, the same components are given the same symbols, and repeated description is omitted. In addition, the drawings are merely schematic, and the proportions of the dimensions of the components relative to each other or the shapes of the components, etc. may differ from the reality.

In addition, reference to subheadings and the like in the following description of the present disclosure is not intended to limit the content or scope of the present disclosure, but is merely intended to serve as a reminder for reading. Such subheadings are neither understood to be used to segment the content of the article, nor should the content under the subheadings be limited only to the scope thereof.

The present disclosure provides an intravascular pressure measurement system, in which the pathological condition of a patient's blood vessel can be determined without the injection of the maximum congestion inducing drug, and the size of the part where the stent needs to be implanted can be more specifically determined.

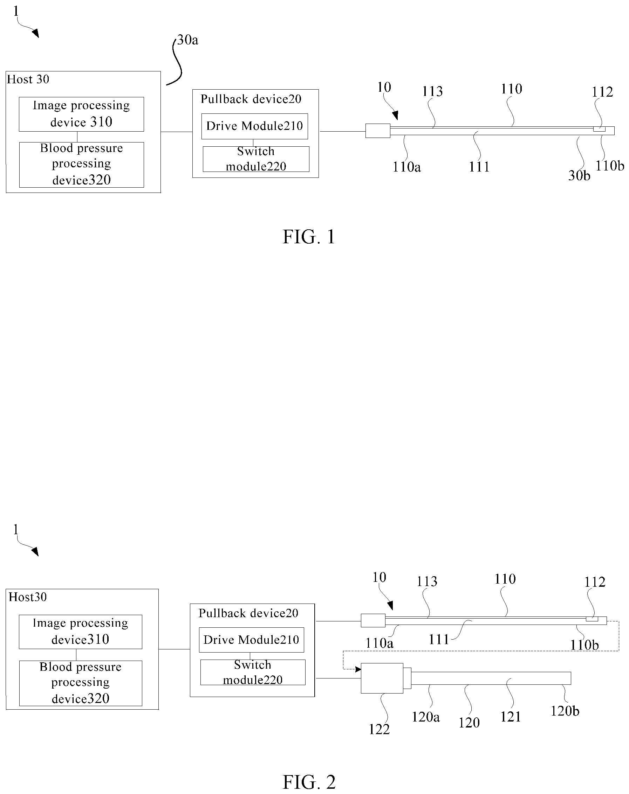

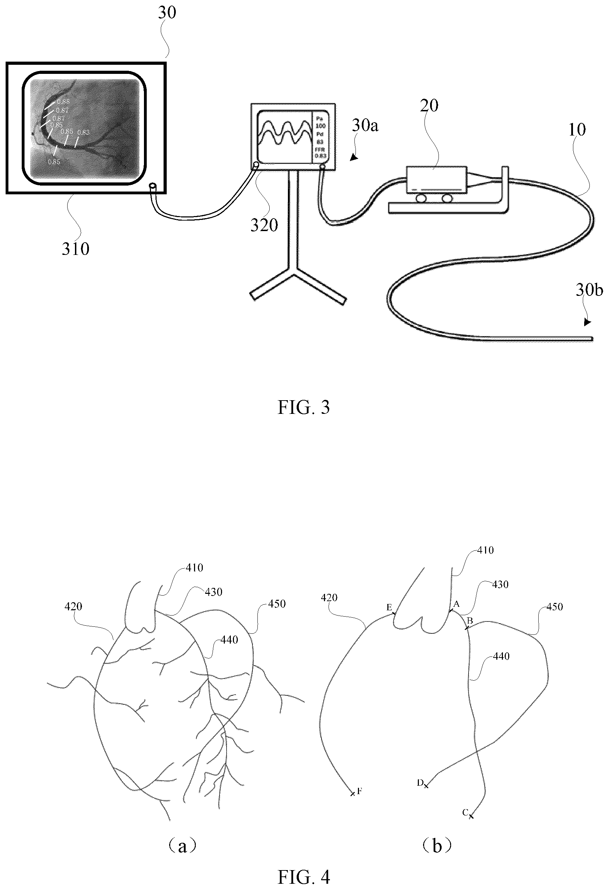

Fig. 1 is a schematic view showing a structure of an intravascular pressure measurement system 1 according to an example of the present disclosure. Fig. 2 is a schematic view showing the structure of an intravascular pressure measurement system 1 according to another example of the present disclosure. Fig. 3 is a schematic view showing an intravascular pressure measurement system 1 according to an example of the present disclosure.

In some examples, as shown in Figs. 1-3, an intravascular pressure measurement system 1 ("system 1" for short) may include a blood pressure measurement device 10, a pullback device 20, and a host 30. The blood pressure measurement device 10 may be connected to the pullback device 20, and the pullback device 20 may be connected to the host 30. The blood pressure measurement device 10 can measure the intravascular blood vessel blood pressure (also referred to as "intravascular pressure", and "pressure" for short) and transmit it to the pullback device 20; that is, the blood pressure measurement device 10 can obtain the intravascular pressure data and transmit it to the pullback device 20. The pullback device 20 may receive pressure data (e.g. the first pressure data described later) from the blood pressure measurement device 10, after which the pressure data may be transmitted to the host 30 for processing.

In some examples, as shown in Fig. 1, one side close to the host 30 (described later) may be a proximal side 30a and one side far away from the host 30 may be a distal side 30b.

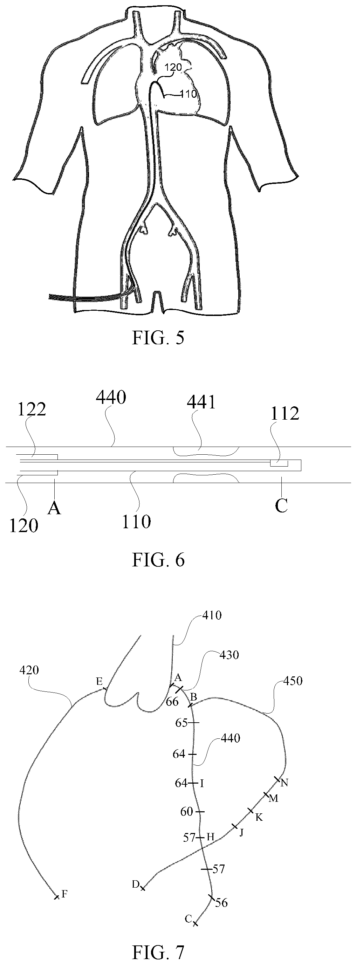

The intravascular pressure measurement system 1 according to the present disclosure can use interventional catheter technology to measure the pressure in a blood vessel to determine a pathological condition of a patient's blood vessel, such as a stenotic lesion 441 (referring to Fig. 6). The intravascular pressure measurement system 1 can be used to measure and process the pressure in the patient's blood vessel to obtain a pressure distribution diagram, a pressure gradient diagram, or a target ratio distribution diagram (described later), can determine the pathological condition of the patient's blood vessel without the injection of the maximum congestion inducing drug, and can more specifically determine the size of the part where the stent needs to be implanted.

In some examples, the blood pressure measurement device 10 may be used to measure the intravascular pressure, i.e. the blood pressure measurement device 10 may obtain the intravascular pressure data.

In some examples, as shown in Fig. 1, the blood pressure measurement device 10 may include a first pressure measurement device. In some examples, the first pressure measurement device has a first pressure sensor that can measure the pressure within the blood vessel and generate pressure data (e.g. the first pressure data described later). In some examples, as shown in Fig. 1, the first pressure measurement device may be a blood pressure measurement catheter 110, which may have a pressure sensor 112 for measuring the pressure in the blood vessel. But the examples of the present disclosure are not limited thereto, the first pressure measurement device may also be other devices for measuring the pressure in the blood vessel, e.g. a medical guide wire with a pressure sensor. In some examples, the first pressure measurement device may be intervened in a patient' s body (see Figs. 5 and 6), the first pressure measurement device may measure the pressure in the blood vessel at a sampling rate via the first pressure sensor and generate the pressure data. In some examples, the pressure data may include a pressure waveform of pressures over time.

In some examples, as shown in Figs. 1 and 2, the blood pressure measurement catheter 110 may be in a form of an elongated tube. In some examples, the blood pressure measurement catheter 110 may have a proximal side 110a proximal to the host 30 and a distal side 110b distal to the host 30, and the blood pressure measurement catheter 110 may have an internal cavity 111. In some examples, as shown in Fig. 1, the blood pressure measurement catheter 110 may be provided with a pressure sensor 112, which may be provided on the distal side 110b of the blood pressure measurement catheter 110. In some examples, the pressure sensor 112 may be provided on the outer wall of the blood pressure measurement catheter 110, in some examples, the pressure sensor 112 may also be provided within the internal cavity 111 of the blood pressure measurement catheter 110, and the internal cavity 111 may have a window matching the pressure sensor 112. In this case, the pressure sensor 112 can be enabled to measure the pressure in the blood vessel to generate the pressure data (e.g. the first pressure data) when the system 1 is working.

In some examples, an X-ray-opaque development ring (not shown) may be provided on at least one side of the first pressure sensor. But the examples of the present disclosure are not limited thereto, in some examples, the first pressure sensor may be provided on an X-ray opaque development ring (e.g. the X-ray opaque development ring may be provided on the distal side 110b of the blood pressure measurement catheter 110 and the pressure sensor 112 may be provided on the X-ray opaque development ring). In this case, the position of the first pressure sensor in the blood vessel can be determined.

In some examples, the first pressure measurement device may be connected (structurally connected) to the pullback device 20, which may control the pullback of the first pressure measurement device within the blood vessel (described later).

In some examples, the first pressure measurement device may be connected (electrically connected) to the pullback device 20. For example, as shown in Fig. 1, a blood pressure measurement catheter 110 may be connected to the pullback device 20. The blood pressure measurement catheter 110 may be provided with a signal path 113, which may be provided in the internal cavity 111 of the blood pressure measurement catheter 110, the signal path 113 may connect the pressure sensor 112 with the pullback device 20. In this case, the pressure data measured by the pressure sensor 112 could be sent to the extracorporeal pullback device 20 via the signal path 113. But the examples of the present disclosure are not limited thereto, in some examples, the first pressure measurement device may be in direct electrical connection with the host 30, thereby enabling the direct transmission of the pressure data (e.g. the first pressure data described later) obtained by the first pressure measurement device to the host 30.

In some examples, the pressure sensor 112 may be a capacitive pressure sensor, a resistive pressure sensor, a fiber optic pressure sensor, etc. Alternatively, the pressure sensor 112 may be a MEMS pressure sensor. For example, the measurement range of the pressure sensor 112 is from about -50mmHg to about + 300mmHg. In some examples, according to the type of pressure sensor 112, the signal path 113 may be a conductive medium, e.g. an electrical lead. Further, in some embodiments, the signal path 113 may also be a wireless communication line, an infrared communication line, or an ultrasonic communication line.

In some examples, as shown in Figs. 1-3, the system 1 may include a pullback device 20. The pullback device 20 may be connected to the blood pressure processing device 320. The pullback device 20 may be provided outside the body.

In some examples, as shown in Fig. 1, the pullback device 20 may include a drive module 210 and a switch module 220.

In some examples, the pullback device 20 may be connected (structurally connected) to the first pressure measurement device. For example, as shown in Fig. 1, the pullback device 20 may be connected to the blood pressure measurement catheter 110. In some examples, the pullback device 20 may have a port matching the proximal side 110a of the blood pressure measurement catheter 110. The pullback device 20 may control the blood pressure measurement catheter 110 for endovascular pullback via the drive module 210. Therefore, the pullback device 20 can control the pullback of the first pressure measurement device within the blood vessel.

In some examples, the pullback device 20 may include a switch module 220 that may be used to control the working state of the drive module 210. For example, if the switch module 220 is in the on state, the drive module 210 can control the automatic pullback of the first pressure measurement device (e.g. the blood pressure measurement catheter 110). If the switch module 220 is in the off state, the drive module 210 is disabled and manual pullback can be performed at this moment, for example, a medical care personnel can control the pullback of the first pressure measurement device (e.g. the blood pressure measurement catheter 110) by pulling the proximal side of the first pressure measurement device or a device connected to the proximal side (e.g. the proximal side 110a of the blood pressure measurement catheter 110 or a device connected to proximal side 110a).

In some examples, the pullback device 20 may be connected (electrically connected) to the first pressure measurement device. In this case, the pullback device 20 can receive and transmit the intravascular pressure data acquired by the first pressure sensor to the host 30 (described later).

In some examples, the pullback device 20 may be configured to control the pullback of the first pressure measurement device (e.g. the blood pressure measurement catheter 110); during the pullback process, the first pressure measurement device may acquire the first pressure data within the blood vessel. For example, during the pullback, the pressure sensor 112 may measure the pressure within the blood vessel, i.e. the pressure sensor 112 may acquire the first pressure data within the blood vessel.

In some examples, the first pressure data may include a first pressure waveform of a plurality of cardiac cycles. In some examples, the first pressure waveform may be a curve of pressures over time. In some examples, the first pressure waveform may be displayed on the host 30 (see the first pressure changing curve described later).

In some examples, the cardiac cycle may include a diastolic phase of diastole and a systolic phase of systole.

Fig. 4 is a angiographic images showing a blood vessel of a preset part according to an example of the present disclosure. Wherein Fig. 4 (a) shows a blood vessel 410, a blood vessel 420, a blood vessel 430, a blood vessel 440, a blood vessel 450, and other small vessels. In the examples of the disclosure, other small vessels may be treated in the same manner as the blood vessel 410, the blood vessel 420, the blood vessel 430, the blood vessel 440, and the blood vessel 450, for convenience of representation, Fig. 4(b) shows a blood vessel of a preset part that does not contain other small vessels. Wherein points A, B, C, D, E, and F are the corresponding positions in the corresponding blood vessels, respectively. For example, the blood vessel 410 may be the aorta, the blood vessel 420 may be the right coronary artery, the blood vessel 430 may be the left main coronary artery, the blood vessel 440 may be the anterior descending coronary artery, and the blood vessel 450 may be the circumflex branch. Fig. 5 is a schematic view showing an intravascular pressure measurement system 1 according to an example of the present disclosure intervening in a human body. Fig. 6 is a cross-sectional view showing an intravascular pressure measurement system 1 according to an example of the present disclosure applied to the blood vessel in Fig. 4. Wherein the blood vessel shown in Fig. 6 may be the blood vessel 440 in Fig. 4, with point A having the same corresponding position in the blood vessel as point A in Fig. 4, and point C having the same corresponding position in the blood vessel as point C in Fig. 4.

In some examples, as shown in Figs. 1-3, the system 1 may include a host 30. The host 30 may be connected to the pullback device 20.

In some examples, the host 30 may receive the pressure data transmitted by the pullback device 20. In some other examples, the first pressure measurement device may be connected to the host 30, and the host 30 may receive pressure data (e.g. the first pressure data) acquired from the first pressure measurement device in the blood vessel.

In some examples, as shown in Figs. 1-3, the host 30 may include an image processing device 310 and a blood pressure processing device 320. In some examples, the image processing device 310 may be connected to the blood pressure processing device 320. In some examples, the blood pressure processing device 320 may be connected to the pullback device 20. In some examples, the blood pressure processing device 320 may receive pressure data (e.g. the first pressure data) transmitted by the pullback device 20. In some other examples, the blood pressure processing device 320 may be connected to the first pressure measurement device, and the blood pressure processing device 320 may receive pressure data (e.g. the first pressure data) acquired from the first pressure measurement device in the blood vessel.

In some examples, the host 30 may be configured to obtain X-ray angiographic images of the blood vessel of a preset part. In particular, prior to the intravascular intervention of the blood pressure measurement device 10, a contrast agent may be injected to the patient and an X-ray angiographic images (e.g. the X-ray angiographic images of the coronary artery in Fig. 4) of a blood vessel of a preset part could be obtained by using the image processing device 310.

In some examples, the image processing device 310 may be an X-ray machine. In some examples, the image processing device 310 may acquire the X-ray angiographic images and generate and transmit a first image signals to the blood pressure processing device 320, and the blood pressure processing device 320 may receive and store the first image signals.

In some examples, the host 30 may be configured to obtain X-ray real-time images, within a blood vessel at a preset part of a first pressure measurement device including a development ring. In some examples, the position of the image processing device 310 may be kept constant, the blood pressure measurement device 10 may be intervened in the blood vessel to be measured (see Figs. 5 and 6), the X-ray real-time images of the first pressure measurement device including the development ring in the blood vessel at the preset part may be obtained via X-ray by the image processing device 310. In some examples, a medical care personnel may push a first pressure sensor (e.g. the pressure sensor 112) to a preset position (e.g. a preset starting point described later) via guidance of X-ray real-time images and X-ray angiographic images. In some examples, during an interventional therapy procedure, the medical professional may first advance the first pressure sensor from a certain position inside a patient body (e.g. at the thigh artery of Fig. 5) along the blood vessel to a preset position within the blood vessel at the pre-set position (e.g. at point C in Figs. 4 and 6). In some examples, the real-time position of the first pressure sensor may be determined through the X-ray real-time images and the X-ray angiographic images during the pullback of the pullback device 20.

In some examples, the image processing device 310 may generate and transmit a second image signal from the obtained X-ray real-time images to the blood pressure processing device 320.

In some examples, the host 30 (e.g. the blood pressure processing device 320) may control whether the pullback device 20 to perform the pullback. In some examples, the host 30 may determine by itself whether the pullback device 20 can be pulled or not. In some examples, the first pressure measurement device may measure the pressure within the blood vessel before the pullback device 20 begins to pull, i.e. the first pressure measurement device may acquire the pressure data within the blood vessel, and the pressure data may include a pressure waveform of the pressures over time, and then the host 30 may receive the pressure data and obtain the pressure waveform. The host 30 can determine whether the obtained intravascular pressure signals are stable or not through the pressure waveform, i.e. determining whether the first pressure measurement device can do stable measurement; if the obtained intravascular pressure signals are stable, the host 30 can control the pullback device 20 to pull. Therefore, the host 30 can control the pullback device 20 to pull.

In some examples, the pressure waveform may include a plurality of cardiac cycles, and the host 30 may determine whether the obtained intravascular pressure signals are stable or not based on the variance of the pressure waveform. But the examples of the present disclosure are not limited thereto, in some examples, the pressure waveform obtained by the host 30 may be compared to a simultaneously obtained electrocardiogram (e.g. by comparing whether the electrocardiogram correlates with the trend of change in the pressure waveform) to determine whether the first pressure measurement device can do stable measurement or not.

In some examples, the host 30 may determine by itself whether the pullback device 20 can be pulled. But the examples of the present disclosure are not limited thereto; in some examples, the medical care personnel may directly control the pullback device 20 to pull by adjusting the host 30.

In some examples, the host 30 may adjust the pullback speed of the pullback device 20, for example, the host 30 or the medical care personnel may adjust the pullback speed of the pullback device 20 via the host 30, the pullback device 20 may work in such manner that the length of pullback of the pullback device 20 during each cardiac cycle time corresponds to a predetermined length of the blood vessel during the pullback, i.e. in each cardiac cycle, the pullback device 20 may correspond to a predetermined length of the blood vessel by controlling the pullback length of the first pressure measurement device in the blood vessel. That is, the host 30 may adjust the pullback speed of the pullback device 20 so that the first pressure measurement device obtains more detailed pressure data of the entire blood vessel, which in turn may improve the spatial resolution of the measured blood vessel in the length direction. In this case, it is able to make the pressure distribution diagram or target ratio distribution diagram obtained later more detailed, whereby the size of the part where the stent needs to be implanted can be determined more specifically. In some examples, the predetermined length of the blood vessel corresponding to the pullback length of each cardiac cycle time may be 0.1-10mm, that is, in each cardiac cycle, the predetermined length of the blood vessel corresponding to pullback length of the first pressure measurement device controlled by the pullback device 20may be 0.1-10mm, for example, the predetermined length of the blood vessel corresponding to the pullback length of each cardiac cycle time may be 0.2mm, 0.5mm, 0.75mm, 1mm, 2.5mm, 3mm, 5mm, or 7.5mm, etc. In some examples, the medical care personnel may set the pullback speed of pullback device 20 by using the host 30. For example, the pullback device 20 is arranged to pull at a constant speed.

In some examples, the host 30 may set a preset starting point and a preset ending point within the blood vessel based on the X-ray angiographic images, and the pullback device 20 may control the first pressure measurement device (i.e. the first pressure sensor) to be pulled back from the preset starting point to the preset ending point. Therefore, the first pressure measurement device can be pulled back from the preset starting point to the preset ending point. In some examples, the medical care personnel may determine that the first pressure sensor is pushed to the preset starting point based on the X-ray angiographic images and the X-ray real-time images. In some examples, the host 30 may determine the pullback of the first pressure measurement sensor to the preset ending point according to the X-ray angiographic images and the X-ray real-time images. In some examples, the host 30 may determine the distance from the development ring on the first pressure measurement device to the preset ending point based on the X-ray angiographic images and the X-ray real-time images, and the host 30 may control the pullback device 20 to stop the pullback if the distance from the development ring on the first pressure measurement device to the preset ending point is equal to or less than the predetermined distance.

In some examples, the host 30 may obtain a measured length of the blood vessel, i.e. the pullback length of the first pressure measurement device, based on the width of the development ring, the X-ray angiographic images and the X-ray real-time images, the preset ending point, and the preset starting point. For example, the host 30 may obtain the ratio of the width of the development ring in the X-ray real-time images to the width of the actual development ring according to the X-ray real-time images and the width of the development ring along the length direction of the blood pressure measurement catheter 110, and obtain the occupation ratio of the width of the development ring in the X-ray real-time image to the blood vessel in the X-ray angiographic images according to the X-ray real-time images and the X-ray angiographic images, thereby being able to calculate the measured length of the blood vessel in the X-ray angiographic images. For example, the host 30 may calculate the length of the blood pressure measurement catheter 110 being pulled back from the preset starting point to the preset ending point.

In some examples, the preset starting point and the preset ending point may be selected by a physician in the X-ray angiographic images. In some examples, the preset ending point may correspond to a bifurcation point of a blood vessel or an end of a blood vessel in X-ray angiographic images. In some examples, the preset starting point may be near the downstream of the blood flow of the blood vessel to be measured and the preset ending point may be near the upstream of the blood flow of the blood vessel to be measured. In some examples, the preset starting point may be distal to the aortic port and the preset ending point may be proximal to the aortic port. It is thereby able to measure the intravascular pressure of the coronary artery. For example, as shown in Fig. 4, the blood pressure measurement device 10 can measure the pressure of a blood vessel 440, the preset starting point can be set at point C, and the preset ending point can be set at point A; the blood pressure measurement device 10 may also measure the pressure of the blood vessel 420 and the preset starting point may be set at point F and the preset ending point may be set at point E.

In some examples, a plurality of blood vessels may be included in the X-ray angiographic images (see Fig. 4). The plurality of blood vessels can be processed in sequence, i.e. the blood vessels in the X-ray angiographic images are processed one by one, whereby the overall hemodynamic condition of the patient's blood vessels can be determined, and it is facilitated for the pressure distribution diagram, target ratio profile, etc. described later to be obtained.

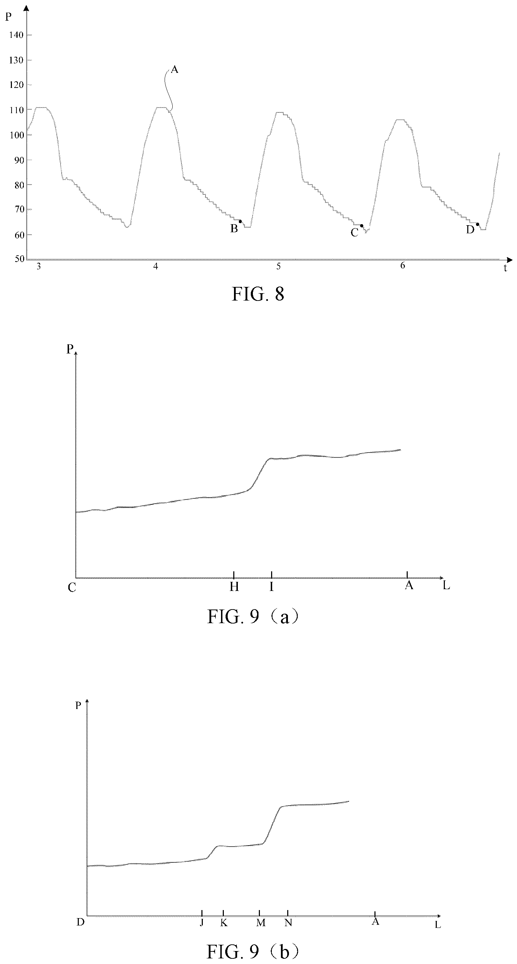

Fig. 7 shows the pressure distribution diagram of the blood vessel of Fig. 4 according to an example of the present disclosure. Fig. 8 is a diagram showing pressure changes for measuring a plurality of cardiac cycles of the blood vessel of Fig. 4 according to an example of the present disclosure. Wherein the corresponding blood vessel of Fig. 8 may be the blood vessel 440 of Fig. 4.

In some examples, the host 30 may obtain a pressure distribution diagram for a blood vessel at a preset part based on the X-ray angiographic images, the X-ray real-time images, and the first pressure data. In some examples, the host 30 may receive the first pressure data (e.g. as shown in Figs. 4 and 6, the first pressure measurement device may measure the pressure of the blood vessel 440), the first pressure data may include a first pressure waveform of a plurality of cardiac cycles, and the host 30 may generate a first pressure changing curve (i.e. a first pressure waveform, see curve A in Fig. 8) of the pressures over time according to the first pressure data. The host 30 may determine each of the cardiac cycles included in the first pressure data according to the first pressure changing curve. In some examples, the host 30 may determine a pressure value corresponding to a particular period (e.g. the end of the diastolic phase, see point B, point C, and point D in Fig. 8) in the diastolic phase of each cardiac cycle as the target pressure value.

In some examples, the host 30 may determine the corresponding position of each target pressure value in the X-ray angiographic images according to the X-ray angiographic images and the X-ray real-time images. In some examples, the host 30 may mark each target pressure value at a corresponding position in the X-ray angiographic images to obtain a pressure distribution diagram (see Fig. 7). In some examples, the medical care personnel or the host 30 may compare each the target pressure value to a first predetermined threshold value for determining a lesion condition of the blood vessel, e.g. whether a stenotic lesion exists or not (e.g. a stenotic lesion is present in the blood vessel 440 as shown in Figs. 6 and 7). In this case, the medical care personnel or the host 30 can determine the lesion of the patient's blood vessel and can more specifically determine the size of the part where a stent needs to be implanted (e.g. a stenotic lesion of the blood vessel 440 between point H and point I as shown in Fig. 7). In some examples, the host 30 may measure a plurality of blood vessels of the X-ray angiographic images separately to obtain pressure distribution diagrams including the plurality of blood vessels of the X-ray angiographic images (not shown). In some examples, the first predetermined threshold value may be set by the medical care personnel themselves. In the present embodiment, the first predetermined threshold value may be obtained by acquiring the pressure data in a blood vessel of a non-diseased person.

In the present embodiment, the first pressure measurement device may have a first pressure sensor that can be used to measure the pressure in the blood vessel, at least one side of the first pressure sensor is provided with an X-ray opaque development ring.

In the present embodiment, the pullback device 20 is connected to the first pressure measurement device and controls the pullback of the first pressure measurement device within the blood vessel, during the pullback procedure, the first pressure sensor acquires the first pressure data within the blood vessel, the pullback device 20 is connected to the host 30, and the host 30 is configured to receive the first pressure data.

In the present embodiment, the host 30 is configured to obtain the X-ray angiographic images of the blood vessel and the X-ray real-time images of the first pressure measurement device including the development ring within the blood vessel, the host 30 obtains a pressure distribution diagram of the blood vessel at the preset part based on the X-ray angiographic images, the X-ray real-time images, and the first pressure data. In this case, the present disclosure enables the determination of a specific lesion condition of a blood vessel of a patient without requiring the injection of maximum congestion inducing drug.

In some examples, the host 30 may set or adjust the sampling rate of the first pressure sensor so that a specific lesion of the patient's blood vessel can be determined, and the lesion area of the blood vessel can be relatively specifically determined. In some examples, the sampling rate of the first pressure sensor may be set directly by the medical care personnel.

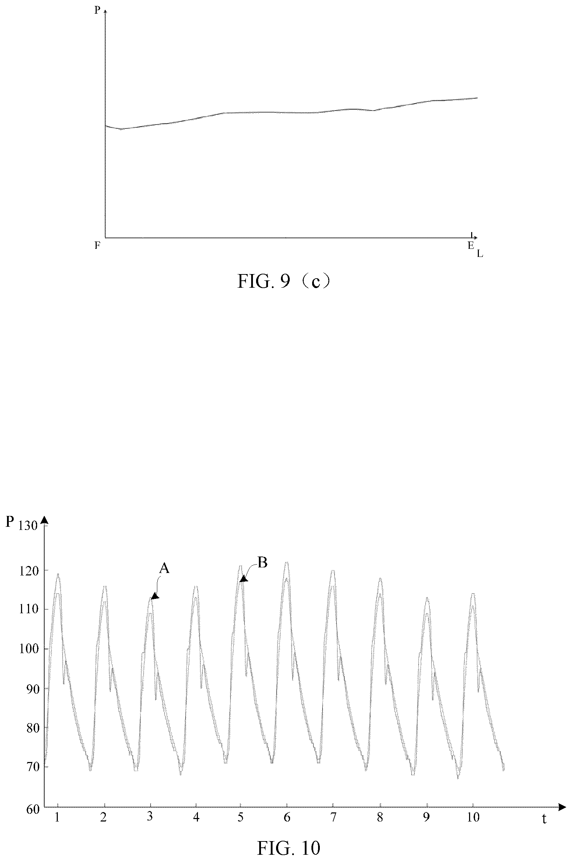

Fig. 9 is a diagram showing the pressure gradient of the blood vessel of Fig. 4 according to an example of the present disclosure. The blood vessel corresponding to Fig. 9 (a) is the blood vessel 440 in Fig. 4, the blood vessel corresponding to Fig. 9 (b) is the blood vessel 450 in Fig. 4, and the blood vessel corresponding to Fig. 9 (c) is the blood vessel 420 in Fig. 4.

In some examples, the host 30 may obtain a pressure gradient diagram along the length direction of the blood vessel at the preset part according to the first pressure data and the pullback speed, and the pressure gradient diagram may be a change diagram of the pressure within the blood vessel as the length direction of the blood vessel changes (see Fig. 9). The pressure gradient diagram can thus be obtained. In some examples, as described above, the host 30 may obtain each of various target pressure values according to the first pressure data. In some examples, the host 30 may obtain the pullback times corresponding to each target pressure value according to the first pressure changing curve. In some examples, the host 30 may determine the length of each target pressure value from the preset ending point according to the set pullback speed and the pullback time corresponding to each target pressure value, and may also determine the position in the X-ray angiographic images corresponding to each target pressure value. In some examples, the pressure gradient diagram may be a curve diagram of changes in the pressure along with the length direction of the blood vessel. In some examples, the host 30 may obtain the measured pressure gradient diagram of the length direction of the blood vessel according to each target pressure value and the length of each target pressure value from the preset ending point. In some examples, the host 30 may obtain a coordinate diagram which may be a discrete point, according to each target pressure value and the length of each target pressure value from a preset ending point. In some examples, the origin of coordinates of the coordinate diagram may be represented as the pressure value corresponding to the preset ending point (e.g. point A in Fig. 4). The horizontal axis may be the length magnitude of the blood vessel, i.e. the distance from the preset ending point, and the vertical axis of the coordinate may be the pressure magnitude. But the examples of the present disclosure are not limited thereto. In some examples, the origin of coordinates of the coordinate diagram may be represented as the pressure value corresponding to a preset starting point (e.g. point C in Fig. 4). The horizontal axis may be the length magnitude of the blood vessel, i.e. the distance from the preset starting point, and the vertical axis of the coordinate may be the pressure magnitude. In some examples, the host 30 can connect discrete points to form a curve to obtain the measured pressure gradient diagram along the length direction of the blood vessel (see Fig. 9). In some examples, the host 30 may measure a plurality of blood vessels of the X-ray angiographic images to obtain the pressure gradient diagram corresponding to each of the plurality of X-ray angiographic images (see Fig. 9). In this case, the medical care personnel can determine the specific lesion condition of the blood vessel of the patient according to the pressure gradient diagram. For example, as shown in Fig. 9 (a), a stenotic lesion is present in blood vessel 440, and within point H and point I, the corresponding position of the stenotic lesion in the blood vessel can be determined (see Fig. 7). As shown in Fig. 9 (b), two stenotic lesions exist in the blood vessel 450, one within point J and point K, and the other one within point M and point N, the corresponding position of the stenotic lesion in the blood vessel may be determined (see Fig. 7). As shown in Fig. 9 (c), no stenotic lesion is present in blood vessel 420.

In some examples, the host 30 may adjust the pullback speed of the pullback device 20 and the host 30 may decrease the pullback speed of the pullback device 20 when the magnitude of the pressure in the pressure gradient diagram decreases with the length direction of the blood vessel by no less than a preset threshold value. The pressure gradient diagram obtained can thus be made more detailed. In some examples, the medical care personnel may set the magnitude of the preset threshold value themselves. In some examples, the preset threshold value may be 1% to 10%, e.g. the preset threshold value may be 2%, 3%, 5%, 7.5%, etc. That is, for example, the preset threshold value may be 5%, i.e. the host 30 may adjust the pullback speed of the pullback device 20 and the host 30 may decrease the pullback speed of the pullback device 20 when the magnitude of the pressure in the pressure gradient diagram decreases with the length direction of the blood vessel by no less than 5%.

In some examples, the host 30 may obtain a measured length of the blood vessel based on the width of the development ring, the X-ray angiographic images, and the X-ray real-time images, and the host 30 may obtain the pullback time of the first pressure measurement device based on the measured length and the pullback speed. The pullback time of the pullback device 20 can thus be obtained.

Fig. 10 is a diagram showing pressure changes for measuring a plurality of cardiac cycles of the blood vessel of Fig. 4 according to another example of the present disclosure. Wherein the corresponding blood vessel of Fig. 10 may be the blood vessel 440 of Fig. 4. Curve A is the first pressure waveform measured by the first pressure sensor and curve B is the second pressure waveform measured by the second pressure sensor.

In some examples, as shown in Fig. 2, the blood pressure measurement device 10 may also include a second pressure measurement device. In some examples, the second pressure measurement device may measure the pressure on the proximal side in the blood vessel (i.e. one side of the blood vessel near the host 30, i.e. one side near the preset ending point) and generate a second pressure data. The second pressure data may include a second pressure waveform of a plurality of cardiac cycles. In some examples, the second pressure measurement device has a second pressure sensor for measuring the intravascular pressure. The second pressure data can thus be obtained. In some examples, the second pressure measurement device may be a guide catheter 120, which may have a pressure sensor 122 for measuring the intravascular pressure. In some examples, the guide catheter 120 may measure the pressure on the proximal side in the blood vessel and generate the pressure data (e.g. the second pressure data). But the examples of the present disclosure are not limited thereto, the second pressure measurement device may also be other devices for measuring the pressure on the proximal side in the blood vessel.

In some examples, as shown in Fig. 2, the second pressure measurement device may be a guide catheter 120, which may have an elongated tubular shape and may have an internal cavity 121. In some examples, the guide catheter 120 has a proximal side 120a proximal to the host 30 and a distal side 120b distal to the host 30.

In some examples, the guide catheter 120 is provided with a pressure sensor 122. In some examples, the second pressure sensor 102 may be an invasive blood pressure sensor that may be directly connected to a port on the proximal side 120a of the guide catheter 120 so as to be provided on the guide catheter 120. For example, the pressure sensor 122 may be provided with a circular interface that matches the tubular structure of the guide catheter 120 for connecting to the guide catheter 120. In that way, the guide catheter 120 and the pressure sensor 122 can match each other more effectively, and the intravascular pressure can be measured more effectively.

In some examples, the pressure sensor 122 may sense the pressure generated by fluid flowing into the internal cavity 121 of the guide catheter 120 from the distal side 120b. In some examples, during the operation of the system 1, the guide catheter 120 may measure the pressure on the proximal side (also referred to as the "first position") in the blood vessel via the pressure sensor 122 at a certain sampling rate and generate the pressure data (e.g. the second pressure data). For example, the guide catheter 120 may be placed in a blood vessel of a human body and the port of the distal side 120b of the guide catheter 120 is placed at a position on the proximal side (e.g. a preset ending point) in the blood vessel, the pressure sensor 122 may be provided outside the body and connected to the port of the proximal side 120a of the guide catheter 120, and the blood at the position on the proximal side in the blood vessel may flow into the guide catheter 120 and may flow from the distal side 120b of the guide catheter 120 to the proximal side 120a of the guide catheter 120 to be sensed by the pressure sensor 122, the pressure sensor 122 can thus obtain the pressure within the internal cavity 121 of the guide catheter 120, i.e. the pressure at a position inside the blood vessel on the proximal side. In this case, the guide catheter 120 can acquire the pressure changing with the cardiac cycle on the proximal side in the blood vessel through the pressure sensor 122 at a certain sampling rate and generate the pressure data.

In other examples, the pressure sensor 122 may be a capacitive pressure sensor, a resistive pressure sensor, a fiber optic pressure sensor, etc., the pressure sensor 122 may be provided on the distal side 120b of the guide catheter 120, e.g. the pressure sensor 122 may be provided on the outer wall of the guide catheter 120. In the operation of the system 1, the guide catheter 120 may be placed within the blood vessel of a human body and the pressure sensor 122 may be placed at a position on the proximal side in the blood vessel. In this case, the pressure sensor 122 may directly sense the pressure at a position on the proximal side in the blood vessel. Therefore, the guide catheter 120 can acquire the pressure changing with the cardiac cycle on the proximal side in the blood vessel through the pressure sensor 122 at a certain sampling rate and generate the pressure data.

In some examples, the second pressure measurement device may be connected to the pullback device 20, and the intravascular pressure data acquired by the second pressure measurement device may be transmitted to the pullback device 20, and then transmitted to the host 30 from the pullback device 20. For example, as shown in Fig. 2, a guide catheter 120 may be connected to the pullback device 20. The pressure sensor 122 may be connected to the pullback device 20 by a transmission wire. In this case, the pressure data acquired by the pressure sensor 122 is transmitted to the pullback device 20 via the transmission wire and then to the host 30. But the examples of the present disclosure are not limited thereto, in some examples, the second pressure measurement device may be connected (electrically connected) to the host 30, and the intravascular pressure data acquired by the second pressure measurement device may be transmitted to the host 30.

In some examples, as shown in Fig. 2, if the first pressure measurement device is a blood pressure measurement catheter 110 and the second pressure measurement device is a guide catheter 120, the diameter of the internal cavity 121 of the guide catheter 120 may be larger than the outer diameter (described later) of the first pressure measurement device (i.e. the blood pressure measurement catheter 110). In some examples, the first pressure measurement device may enter the internal cavity 121 from the proximal side 120a of the guide catheter 120 (see Figs. 1 and 3). In this case, during the operation of the system 1, the blood pressure measurement catheter 110 can be provided in the internal cavity 121 which facilitates the movement of the blood pressure measurement catheter 110 relative to the guide catheter 120, and the guide catheter 120 can be kept stationary.

In some examples, the first pressure measurement device may be deeper into the patient relative to the second pressure measurement device, and the first pressure sensor (e.g. pressure sensor 112) may measure the intravascular pressure deeper into the human body (e.g. a preset starting point) relative to the second pressure sensor (e.g. the pressure sensor 122).

In some examples, the second pressure sensor may measure the pressure on the proximal side (i.e. the first position) in the blood vessel, and the medical care personnel may adjust the position measured by the second pressure sensor (i.e. the first position). In some examples, the preset ending point may be the first position, and the second pressure sensor may measure the pressure at the preset ending point and generate the pressure data. For example, the port of the distal side 120b of the guide catheter 120 may be provided with an X-ray opaque development ring, and the port of the distal side 120b of the guide catheter 120 may be set as the preset ending point.

In some examples, the blood pressure measurement catheter 110 and the guide catheter 120 may each enter or exit the patient as separate devices. In this case, the medical staff can independently control the blood pressure measurement catheter 110 and the guide catheter 100. For example, while the system 1 is in operation, the medical care personnel may control the blood pressure measurement catheter 110 to move relatively to the guide catheter 120 (e.g. advance deep into or be pulled back deep from a blood vessel in the patient), and the guide catheter 120 may remain stationary.

In some examples, the second pressure sensor and the first pressure sensor measure the pressures within respective blood vessels simultaneously at the same sampling rate and generate respective pressure data.

In some examples, the blood pressure measurement device 10 may include a second pressure measurement device, during the pullback procedure, the first pressure measurement device may measure the pressure in the blood vessel and generate the first pressure data, and the second pressure measurement device may measure the pressure on the proximal side in the blood vessel and generate the second pressure data. In some examples, the second pressure data may include a second pressure waveform of a plurality of cardiac cycles. In some examples, the second pressure waveform may be a curve of the pressures over time. In some examples, the second pressure waveform may be displayed on the host 30 (see curve B in Fig. 10).

In some examples, the host 30 may receive the second pressure data and the first pressure data, and the host 30 may be configured to synchronize the first pressure waveform and the second pressure waveform such that the moment when the peak value of the first pressure waveform appears in the cardiac cycle is the same as the moment when the peak value of the second pressure waveform appears. That is, the host 30 may make, in the same cardiac cycle, the moment when the peak value of the first pressure waveform appears is the same as the moment when the peak value of the second pressure waveform appears. The first pressure waveform and the second pressure waveform can thus be synchronized to facilitate the subsequent acquisition of an accurate target ratio.

In some examples, the host 30 may receive the second pressure data and the first pressure data, and the host 30 may be configured to discard apparently invalid signals. For example, the maximum value (or the average value, the peak-to-peak value) of the first pressure signals is greater than the 125% of the maximum value (or the average value, the peak-to-peak value) of the second pressure waveform.

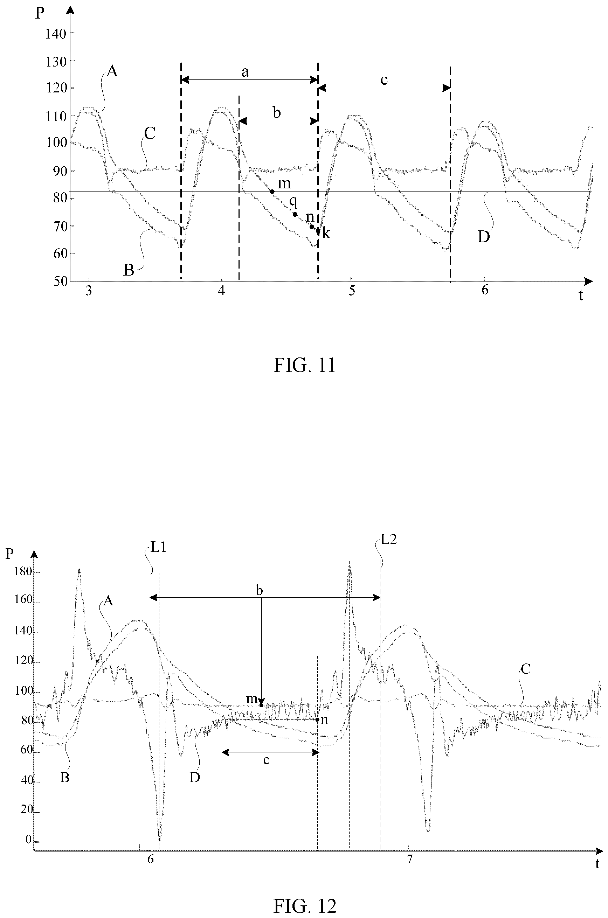

Fig. 11 is a pressure waveform diagram showing a plurality of cardiac cycles of Fig. 10 according to an example of the present disclosure. Curve A in Fig. 11 is a first pressure waveform, curve B is a second pressure waveform, curve C is a curve of the pressure ratio of the pressure value of the first pressure waveform to the pressure value of the corresponding second pressure waveform (wherein curve C shows a pressure ratio that is a hundred times the actually calculated pressure ratio), and curve D is a curve of the pressure mean value of the pressure value of the second pressure waveform. Interval a and interval c are the entire cardiac cycle, interval b is the diastolic phase of the cardiac cycle, point m is the first intersection point (described later), point n is the minimum value of the second pressure waveform in the descending time where the first intersection point is located , point k is the minimum value of the second pressure waveform, and point q corresponds to the time at 80% of the cardiac cycle.

Fig. 12 is a pressure waveform diagram showing one cardiac cycle of Fig. 10 according to another example of the present disclosure. Curve A in Fig. 12 is a first pressure waveform, curve B is a second pressure waveform, curve C is a curve of the pressure ratio of the pressure value of the first pressure waveform to the pressure value of the corresponding second pressure waveform (wherein curve C shows a pressure ratio that is a hundred times the actually calculated pressure ratio), and curve D is the curve of the derivative of the pressure value of the first pressure waveform with respect to time. The line L1 corresponds to a first midpoint moment, the line L2 corresponds to a second midpoint moment, the interval b is a moment period from the first midpoint moment to the second midpoint moment, the point m is a pressure ratio corresponding to a midpoint moment of the interval b, and the point n is a target derivative value.

In some examples, the host 30 may generate a waveform diagram with the received pressure data for display on a display screen in the form of a waveform (see Figs. 8 and 10). Wherein the vertical axis of the waveform diagram may be the magnitude of the pressure (i.e. the pressure value), and the horizontal axis of the waveform diagram may be the time axis.

In some examples, the host 30 may receive the second pressure data and the first pressure data, and the host 30 may obtain a plurality of target ratios based on the second pressure data and the first pressure data. In some examples, the host 30 may obtain a target ratio distribution diagram of a blood vessel at a preset part based on the plurality of target ratios, X-ray angiographic images, and X-ray real-time images. The target ratio distribution diagram can thus be obtained.

In some examples, the host 30 may receive the second pressure data and the first pressure data, and the host 30 may obtain a plurality of target ratios based on the second pressure data and the first pressure data.

In some examples, the host 30 may be configured to determine a plurality of cardiac cycles (see intervals a and c of Fig. 11) based on the first pressure waveform or the second pressure waveform, calculate a plurality of pressure ratios of the pressure values of the first pressure waveform and the corresponding pressure values of the second pressure waveform, and sequentially select an average value of a predetermined number of pressure ratios, or a minimum value of the pressure ratios corresponding to the diastolic phase in each cardiac cycle in the order from small to large from pressure ratios corresponding to each cardiac cycle, , as the target ratio. The target ratio can thus be obtained. In some examples, the host 30 may receive the second pressure data and the first pressure data, i.e. the host 30 may obtain the first pressure waveform (see curve A in Fig. 11) and the second pressure waveform (see curve B in Fig. 11), the host 30 may calculate a plurality of pressure ratios of the pressure values of the first pressure waveform to pressure values of the corresponding second pressure waveform (e.g. pressure values of the first pressure waveform and the second pressure waveform at the same moment being corresponding pressure values), and the host 30 may determine a plurality of cardiac cycles based on the first pressure waveform or the second pressure waveform. In some examples, the host 30 may select an average value of a predetermined number (e.g. 3-5) of pressure ratios in the order from small to large for the pressure ratios (see curve C in Fig. 11) corresponding to each cardiac cycle (e.g. interval a or interval c in Fig. 11) as the target ratio. In some examples, the host 30 may use the minimum value of the pressure ratios (see point k) corresponding to the diastolic phase (e.g. interval b in Fig. 11) in each cardiac cycle as the target ratio.

In some examples, the host 30 may be configured to determine a plurality of cardiac cycles based on the first pressure waveform or the second pressure waveform, calculate a curve of the pressure ratios of the pressure values of the first pressure waveform to the pressure values of the corresponding second pressure waveform as a first changing curve, and obtain the curve of pressure mean value of the second pressure waveform based on the second pressure waveform as a second changing curve, a point at which the second pressure waveform in descending time intersects the second changing curve in each cardiac cycle is a first intersection point corresponding to the cardiac cycle, the pressure ratio corresponding to the minimum value of the second pressure waveform in descending time in each cardiac cycle, or the pressure ratio corresponding to the midpoint of the time period from the first intersection point to the minimum value of the second pressure waveform in each cardiac cycle, or the average value of the corresponding pressure ratios in the time period from the first intersection point in each cardiac cycle to 80% of the cardiac cycle, or the average value of the corresponding pressure ratios in the time period from the first intersection point to the minimum value of the second pressure waveform in each cardiac cycle is taken as target ratio. The target ratio can thus be obtained.

In some examples, the host 30 may receive the second pressure data and the first pressure data, i.e. the host 30 may obtain a first pressure waveform (see curve A in Fig. 11) and a second pressure waveform (see curve B in Fig. 11), the host 30 may calculate a curve of a plurality of pressure ratios of the pressure values of the first pressure waveform to the corresponding pressure values of the second pressure waveform as the first changing curve (e.g. curve C in Fig. 11), and the host 30 may, based on the second pressure waveform, obtain a curve of the mean value of the pressures of the second pressure waveform as a second changing curve (e.g. curve D in Fig. 11), wherein the point at which the second pressure waveform in the descending time intersects with the second changing curve within each cardiac cycle may serve as the corresponding first intersection point of the cardiac cycle (e.g. point m in Fig. 11). In some examples, the host 30 may take the pressure ratio corresponding to the minimum value (e.g. point n in Fig. 11) of the second pressure waveform in the descending time where the first intersection point is located (e.g. curve B in Fig. 11) in each cardiac cycle as the target ratio. In some examples, the host 30 may take the pressure ratio corresponding to the midpoint of the time period from the first intersection point to the minimum value of the second pressure waveform (e.g. point k in Fig. 11) within each cardiac cycle as the target ratio. In some examples, the host 30 may take the average value of the corresponding pressure ratios over the time period from the first intersection point to 80% of the cardiac cycle (e.g. point q in Fig. 11) in each cardiac cycle as the target ratio. In some examples, the host 30 may take the average value of the corresponding pressure ratios over the time period from the first intersection point to the minimum value of the second pressure waveform over each cardiac cycle as the target ratio.

In some examples, the host 30 may be configured to determine a plurality of cardiac cycles based on the first pressure waveform or the second pressure waveform, calculate a curve of the pressure ratio of the pressure value of the first pressure waveform to the pressure value of the corresponding second pressure waveform as a first changing curve (see curve C in Fig. 12), and obtain the curve of the derivative of the pressure value of the first pressure waveform with respect to time based on the first pressure waveform as the third changing curve (see curve D in Fig. 12), the moment corresponding to the midpoint of the time period from the maximum value of the first pressure waveform to the minimum value of the third changing curve in any cardiac cycle can be the first midpoint moment corresponding to the cardiac cycle, and the moment corresponding to the midpoint of the time period from the maximum value of the first pressure waveform to the maximum value of the third changing curve in any cardiac cycle can be the second midpoint moment corresponding to the cardiac cycle, the pressure ratio corresponding to the midpoint of the time period from the first midpoint moment to the second midpoint moment in each cardiac cycle, or the average value of the corresponding pressure ratios in the time period from the first midpoint moment to the second midpoint moment in each cardiac cycle from the first to the last occurrence of the target derivative value can be taken as the target ratio, wherein the target derivative value may be the derivative value that occurs most frequently in the third changing curve during the time period from the first midpoint moment to the second midpoint moment in the cardiac cycle. The target ratio can thus be obtained.

In some examples, host 30 may be configured to determine a plurality of cardiac cycles based on the first pressure waveform or the second pressure waveform, calculate a curve of the pressure ratio of the pressure value of the first pressure waveform to the pressure value of the corresponding second pressure waveform as a first changing curve (see curve C in Fig. 12), and obtain a curve of the derivative of the pressure value of the first pressure waveform with respect to time based on the first pressure waveform as the third changing curve (see curve D in Fig. 12). In some examples, the moment corresponding to the midpoint of the time period from the maximum value of the first pressure waveform to the minimum value of the third changing curve in any cardiac cycle may be the first midpoint moment corresponding to that cardiac cycle. In some examples, the moment corresponding to the midpoint of the time period from the maximum value of the first pressure waveform to the maximum value of the third changing curve in any cardiac cycle may be the second midpoint moment corresponding to that cardiac cycle. In some examples, the host 30 may take the pressure ratio (e.g. point m in Fig. 12) corresponding to the midpoint of a time period (e.g. interval b in Fig. 12) from the first midpoint moment to the second midpoint moment in each cardiac cycle as the target ratio. In some examples, the host 30 may take the average value of the corresponding pressure ratios over the time period from the first occurrence to the last occurrence (e.g. interval c in Fig. 12) of the target derivative value (e.g. point n in Fig. 12) over the time period from the first midpoint moment to the second midpoint moment in each cardiac cycle as the target ratio.

In the present disclosure, the host 30 may receive the first pressure data and the second pressure data and obtain the target ratio, as described above. In some examples, the host 30 may select several cardiac cycles to process to obtain a plurality of target ratios. In some examples, the host 30 may process each cardiac cycle in sequence to obtain a plurality of target ratios. In some examples, the host 30 or the medical care personnel may compare the obtained target ratios with a second predetermined threshold value to determine the lesion condition of the patient's blood vessel, thereby enabling the lesion condition of the patient's blood vessel to be determined without requiring the injection of maximum congestion inducing drug. In some examples, the second predetermined threshold value may be set by the medical care personnel themselves. In the present embodiment, the second predetermined threshold value may be obtained by acquiring the pressure data in a blood vessel of a non-diseased person.

In some examples, the host 30 can obtain the corresponding position of the first pressure sensor (i.e. the obtained first pressure waveform) in the X-ray angiographic images in each cardiac cycle based on the pullback speed, the corresponding time of each cardiac cycle, and the X-ray real-time images and the X-ray angiographic images, thereby being able to determine the corresponding position of each target ratio in the X-ray angiographic images. In some examples, the host 30 may mark the obtained target ratio at a corresponding position in the X-ray angiographic images. In some examples, the corresponding position of the target ratio in the X-ray angiographic images may not be one fixed point and may have a certain length, and the host 30 may mark the target ratio at any position in the length (e.g. the midpoint of the length). In this case, it is able to obtain a target ratio distribution diagram of the blood vessel at a preset part (see Fig. 3). Therefore, the specific lesions of each blood vessel can be determined, and the size of the part where a stent needs to be implanted can be determined relatively specifically.

In some examples, the system 1 may perform pressure measurements on each blood vessel in X-ray angiographic images and employ the host 30 to process so as to determine the specific lesions of each blood vessel.

In some examples, the pullback device 20 may be configured to be in a stopped state, the host 30 may not control the pullback device 20 to pull (i.e. the pullback device 20 is not required), the first pressure sensor may measure the pressure at a predetermined position (e.g. a preset starting point) with a certain sampling rate and generate a third pressure data, and the second pressure sensor may measure the pressure at the first position (e.g. a preset ending point) with a certain sampling rate and generate a fourth pressure data. The host 30 may receive and process the third pressure data and the fourth pressure data to obtain a target ratio of the third pressure data to the fourth pressure data. In some examples, the third pressure data may be analogized to the first pressure data, the fourth pressure data may be analogized to the second pressure data, and the host 30 may process any complete cardiac cycle to obtain a target ratio of the third pressure data to the fourth pressure data. In some examples, the host 30 or the medical care personnel may compare the obtained target ratios with a second predetermined threshold value to determine the lesion condition of the patient's blood vessel, thereby enabling the lesion condition of the patient's blood vessel to be determined without requiring the injection of maximum congestion inducing drug. Although the present disclosure has been specifically described above with reference to the accompanying drawings and embodiments, it could be understood that the above description does not limit the present disclosure in any form. Modifications and variations of the present disclosure, as required, may be made by those skilled in the art without departing from the true spirit and scope of the present disclosure, and shall fall into the scope of the present disclosure.

## Claims

1. An intravascular pressure measurement system **characterized by** comprising:
a first pressure measurement device having a first pressure sensor for measuring pressure within a blood vessel and an X-ray opaque development ring provided on at least one side of the first pressure sensor;
a pullback device connected to the first pressure measurement device and controlling the first pressure measurement device to be pulled back within the blood vessel, wherein during the pullback procedure, the first pressure sensor acquires a first pressure data within the blood vessel, the first pressure data comprising first pressure waveforms of a plurality of cardiac cycles; and
a host configured to obtain an X-ray angiographic images of a blood vessel at a preset part and X-ray real-time images of the first pressure measurement device comprising the development ring in the blood vessel at the preset part, the host is connected to the pullback device and configured to receive the first pressure data, and the host obtains a pressure distribution diagram of the blood vessel at the preset part based on the X-ray angiographic images, the X-ray real-time images, and the first pressure data.

2. The intravascular pressure measurement system according to claim 1, **characterized in that**:
the host sets a preset starting point and a preset ending point in the blood vessel based on the X-ray angiographic images, and the pullback device controls the first pressure measurement device to be pulled back from the preset starting point to the preset ending point.

3. The intravascular pressure measurement system according to claim 2, **characterized in that**:
the preset starting point is far away from an aortic port and the preset ending point is close to the aortic port.

4. The intravascular pressure measurement system according to claim 1, **characterized in that**:
the host determines whether pressure signals in the blood vessel are stable or not based on the first pressure waveform obtained by the first pressure measurement device, and controls the pullback device to pull when the pressure signals are stable.

5. The intravascular pressure measurement system according to claim 1, **characterized by** further comprising
a second pressure measurement device, wherein the second pressure measurement device has a second pressure sensor for measuring a pressure of a proximal side in the blood vessel and generating a second pressure data, the second pressure data comprising a second pressure waveform of a plurality of cardiac cycles.

6. The intravascular pressure measurement system according to claim 5, **characterized in that**
the host receives the second pressure data, and the host obtains a plurality of target ratios based on the first pressure data and the second pressure data, and obtains a target ratio distribution diagram of a blood vessel at the preset part based on the X-ray angiographic images, the X-ray real-time images, and the a plurality of target ratios.

7. The intravascular pressure measurement system according to claim 6, **characterized in that**
the host is configured to determine a plurality of cardiac cycles based on the first pressure waveform or the second pressure waveform, calculate a plurality of pressure ratios of pressure values of the first pressure waveform to the pressure values of the corresponding second pressure waveform, and sequentially select an average value of a predetermined number of pressure ratios in an order from small to large from pressure ratios corresponding to each cardiac cycle , or a minimum value of the pressure ratios corresponding to a diastolic phase in each cardiac cycle as the target ratio.

8. The intravascular pressure measurement system according to claim 6, **characterized in that**
the host is configured to determine a plurality of cardiac cycles based on the first pressure waveform or the second pressure waveform, calculate a curve of a pressure ratio of a pressure value of the first pressure waveform to a pressure value of a corresponding second pressure waveform as a first changing curve, and obtain the curve of a mean value of the pressure of the second pressure waveform based on the second pressure waveform as a second changing curve, wherein a point at which the second pressure waveform in a descending time intersects the second changing curve in each cardiac cycle is a first intersection point corresponding to the cardiac cycle, the pressure ratio corresponding to a minimum value of the second pressure waveform in the descending time in each cardiac cycle, or the pressure ratio corresponding to a midpoint of a time period from the first intersection point to the minimum value of the second pressure waveform in the cardiac cycle, or an average value of corresponding pressure ratios in the time period from the first intersection point in the cardiac cycle to 80% of the cardiac cycle, or the average value of the corresponding pressure ratios in the time period from the first intersection point to the minimum value of the second pressure waveform in the cardiac cycle is taken as target ratio.

9. The intravascular pressure measurement system according to claim 6, **characterized in that**
the host is configured to determine a plurality of cardiac cycles based on the first pressure waveform or the second pressure waveform, calculate a curve of pressure ratios of pressure values of the first pressure waveform to pressure values of a corresponding second pressure waveform as a first changing curve, and obtain the curve of derivatives of the pressure values of the first pressure waveform with respect to time based on the first pressure waveform as a third changing curve, wherein a moment corresponding to a midpoint of the time period from a maximum value of the first pressure waveform to a minimum value of the third changing curve in any cardiac cycle is a first midpoint moment, a moment corresponding to a midpoint of a time period from a maximum value of the first pressure waveform to a maximum value of the third changing curve in any cardiac cycle is a second midpoint moment, and a pressure ratio corresponding to a midpoint of a time period from the first midpoint moment to the second midpoint moment in the cardiac cycle, or an average value of the corresponding pressure ratios in a time period from the first midpoint moment to the second midpoint moment in the cardiac cycle from a first to a last occurrence of a target derivative value is taken as the target ratio, wherein the target derivative value is a derivative value that occurs most frequently in the third changing curve during a time period from the first midpoint moment to the second midpoint moment in the cardiac cycle during a time period from the first midpoint moment to the second midpoint moment in the cardiac cycle.

10. The intravascular pressure measurement system according to claim 1, **characterized in that**
the host adjusts a pullback speed of the pullback device.

11. The intravascular pressure measurement system according to claim 10, **characterized in that**
the host obtains a pressure gradient diagram of a length direction of a blood vessel at the preset part based on the first pressure data and the pullback speed, the pressure gradient diagram is a change diagram of the pressures within the blood vessel as the length direction of the blood vessel changes.

12. The intravascular pressure measurement system according to claim 10, **characterized in that**
the host adjusts the pullback speed of the pullback device to reduce the pullback speed of the pullback device when a magnitude of the pressure in the pressure gradient diagram decreases with the length direction of the blood vessel and the the pressure is no less than a preset threshold value.

13. The intravascular pressure measurement system according to claim 6, **characterized in that**
the host is configured to synchronize the first pressure waveform and the second pressure waveform such that a moment when a peak value of the first pressure waveform appears in the cardiac cycle is the same as the moment when the peak value of the second pressure waveform appears.
